(19) Europäisches Patentamt
European Patent Office
Office européen des brevets

(11) Publication number: 0 273 132 A2

(12) # EUROPEAN PATENT APPLICATION

(21) Application number: 87115749.1

(22) Date of filing: 27.10.87

(51) Int. Cl.4: C07C 125/073, C07C 119/042, C08L 33/14

(30) Priority: 03.11.86 US 926054

(43) Date of publication of application: 06.07.88 Bulletin 88/27

(84) Designated Contracting States: AT BE CH DE FR GB IT LI NL SE

(71) Applicant: AMERICAN CYANAMID COMPANY
1937 West Main Street P.O. Box 60
Stamford Connecticut 06904-0060(US)

(72) Inventor: Alexanian, Vazken Arsen
11 Renshaw Road
Darien Connecticut(US)
Inventor: Fiala, Robert
24 Guinea Road
Cos Cob Connecticut 06807(US)

(74) Representative: Wächtershäuser, Günter, Dr.
Tal 29
D-8000 München 2(DE)

(54) **Preparation of a novel aliphatic diisocyanate from styrene.**

(57) Novel cycloaliphaticalkyl dicarbamates are prepared by hydrogenating aralkyl carbamates, and these are subsequently thermally cracked to novel cycloaliphatic alkyl diisocyanates.



Xerox Copy Centre

# NOVEL CYCLOALIPHATICALKYL DIURETHANES AND DIISOCYANATES AND PROCESS FOR THEIR PREPARATION

## FIELD OF THE INVENTION

The present invention relates to a novel process for preparation of novel cycloaliphaticalkyl carbamates and diisocyanates from starting materials comprising monovinyl aromatic compounds. The cycloaliphaticalkyl diisocyanates can be reacted with diols and polyols to produce polyurethanes for coating and molding applications.

## BACKGROUND OF THE INVENTION

Conrad, M. and Hock, K., Formaldehydderivate der Urethane, Berichte, 36, 2206 (1903) disclose the condensation reaction of formaldehyde and the ethyl ester of carbamic acid or urethane to produce methylene diurethane (MEDU), a compound of the formula

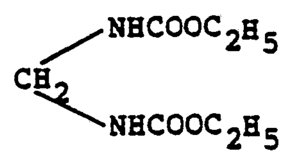

In Angew. Chem. , 74(21), 866 (1962), Mueller and Merten teach an addition reaction of MEDU and styrene in the presence of a Lewis acid, e.g., boron trifluoride, to produce a compound of the formula

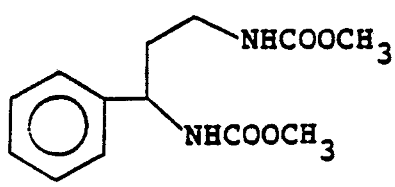

This compound is hydrolyzed by the addition of water and base to the diamine derivative which is phosgenated to yield an aralkyl diisocyanate of the formula

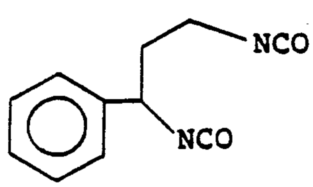

The use of such processes are tedious and require the employment of the toxic gas phosgene and the disposal of corrosive hydrogen chloride as a by product. Aromatic-containing diisocyanates are not particularly light-fast when cured into polyurethanes and they do not have low toxicity.

Alexanian et al., U.S. 4,379,767, disclose a process for the production of tertiary benzyl isocyanates by reaction of the corresponding olefin, e.g., alpha-methyl styrene with a carbamoyl halide to form the benzyl halide, which is subsequently reacted with an excess of isocyanic acid to form the isocyanate. Such a process requires multiple steps, and the end product is aromatic in character.

It has been discovered now that wholly aliphatic diisocyanate compounds can be prepared using inexpensive starting materials, for example, styrene, formaldehyde and methyl carbamate to produce a

mixture of aralkyl carbamates which can be hydrogenated to produce new wholly aliphatic carbamates. These novel carbamates can in turn, be cracked to yield new and useful diisocyanate cycloaliphatic alkyl derivatives, heretofore unobtainable in this way from the prior art. The novel products lack many of the disadvantages of those of the prior art. In particular, they have high reactivity, light stability, and selectivity.

## SUMMARY OF THE INVENTION

According to the present invention, there is provided a process for the preparation of a diurethane of the formula:

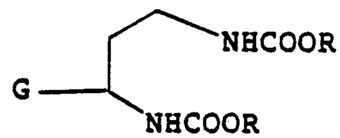

(I)

(I)

wherein G is selected from

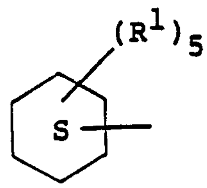

and

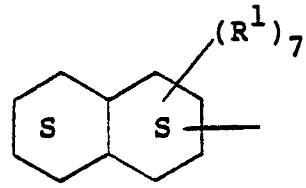

R is selected from alkyl of from about 1 to about 30 carbon atoms, preferably 1 to 18, and $R^1$ is selected from hydrogen and alkyl of from about 1 to about 30 carbon atoms, preferably 1 to 3, said process comprising:

(a) reacting

(i) a vinyl aromatic hydrocarbon of the formula

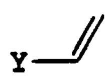

(II)

wherein Y is selected from

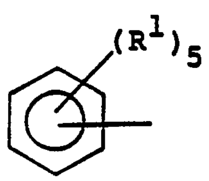

and

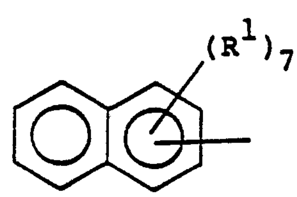

,

with

(ii) a methylene bis-alkylcarbamate of the formula

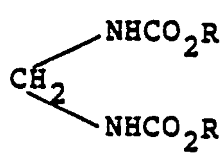

(III)

wherein R is as above defined, in the presence of

(iii) an effective catalytic amount of an acid at a temperature of from about 40°C to about 150°C until formation of a diurethane compound of the formula

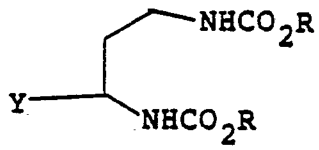

(IV)

wherein R and Y are as defined above, is substantially complete, and

(b) catalytically hydrogenating the compound of formula (IV) until formation of the diurethane compound of formula (I) is substantially complete.

Also contemplated by the present invention are urethane compounds of the formula:

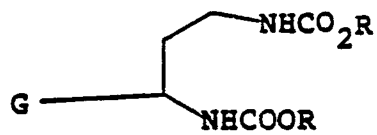

(I)

wherein G is selected from

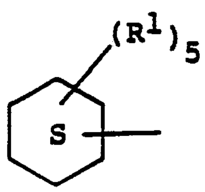

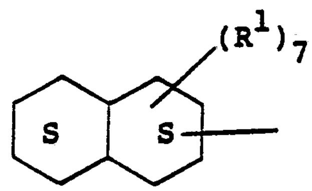

R is alkyl of from about 1 to about 30 carbon atoms, preferably 1 to 18, and $R^1$ is hydrogen or alkyl of from about 1 to about 30 carbon atoms, preferably 1 to 3.

Also contemplated herein is a process for the preparation of isocyanate compounds of the formula:

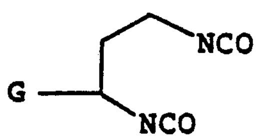

(V)

wherein G is selected from

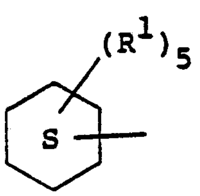

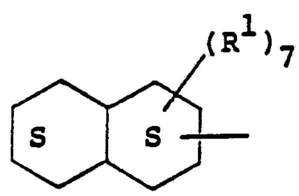

and $R^1$ is hydrogen or alkyl of from about 1 to about 30 carbon atoms, preferably 1 to 3, said process comprising heating a diurethane of the formula:

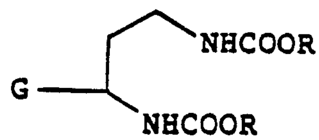

(I)

wherein G is as above defined and R is alkyl of from about 1 to about 30 carbon atoms, preferably 1 to 18, at a temperature of from about 150°C to about 700°C until formation of said diisocyanate compound of formula V is substantially complete.

Also provided in accordance with this invention are diisocyanate compounds of the formula:

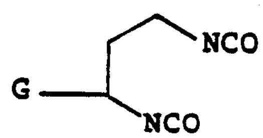

(V)

wherein G is selected from

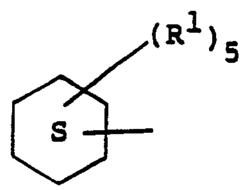

and

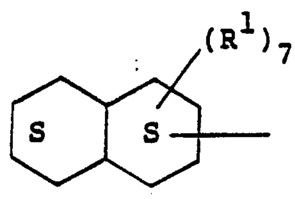

wherein $R^1$ is hydrogen or alkyl of from about 1 to about 30 carbon atoms, preferably 1 to 3.

Among the features of this invention are curable compositions comprising:

(i) active hydrogen compounds, especially polyols, preferably hydroxy functional polyacrylates; and

(ii) the novel diisocyanates above defined.

Other objects and advantages of the present invention will become apparent from the following detailed description of the invention taken in conjunction with the illustrative working examples.

DETAILED DESCRIPTION OF THE INVENTION

The monovinyl aromatic compounds of formula (II)

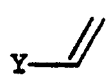

can vary widely in type. Y can comprise mono and bicyclic aromatic groups of the formulae

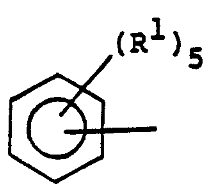

and

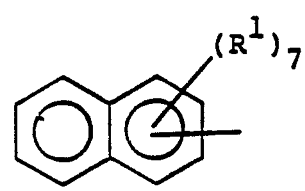

wherein $R^1$ is hydrogen or alkyl of from about 1 to about 30, preferably 1 to 3, carbon atoms, e.g., methyl, ethyl, n-propyl, i-propyl, octadecyl, and triacontyl. Illustrative such compounds of formula II are styrene, vinyl toluene, the vinyl xylenes, ethyl vinyl benzene, vinyl mesitylene, vinyl naphthalene, methyl vinyl

naphthalene and the like. Preferably the vinyl aromatic compound will be styrene, i.e., a compound of the formula

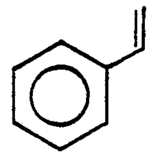

All of the vinyl aromatic compounds can be made in known ways, and many are commercially available.

The other starting materials, namely, the compounds

of formula (III)

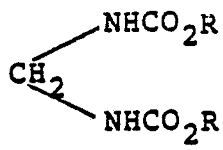

(III)

wherein R is alkyl of from 1 to 30, preferably 1 to 18, car bon atoms, straight chain and branched, e.g., methyl, ethyl, propyl, 2-ethylhexyl, n-octadecyl, and the like, also can be produced in known ways, e.g., by the technique described by Conrad et al. above, using suitably substituted alkyl esters of carbamic acid. Instead of gaseous formaldehyde or aqueous formaldehyde, a formaldehyde precursor, such as trioxymethylene, paraform, formcel, and the like can be used. Preferably in the methylene bis-alkyl carbamate (III) R is methyl.

To carry out the condensation between (II) and (III) it is necessary to heat the components in the presence of an acidic catalyst. In accordance with this invention the preparation of the aralkylurethane ester takes place at temperatures from 40°C up to 150°C in the presence of an acid such as sulfuric acid, toluene sulfonic acid, dodecyl benzene sulfonic acid, hydrocarbon sulfate esters, hydrochloric acid, boron trifluroide, and other Lewis and Bronstead acids. The reaction can take place in the absence of solvent or in the presence of solvents such as methylene chloride, toluene, xylene, chlorobenzene, and the like.

As will be obvious to those skilled in this art, the alkylene-bis-alkylcarbamate (III) can be generated in situ either by pre-reacting formaldehyde or a formaldehyde precursor with the alkyl carbamate or by reacting a mixture comprising formaldehyde, the alkyl carbamate and the vinyl aromatic compound.

In any event the proportion of methylene bis carbamic acid ester to vinyl aromatic is near to stoichiometric. If bis-carbamate is in substantial excess there is a tendency to produce appreciable amounts of a cyclic byproduct. Such byproduct can be carried through the process without adverse effect, but its removal later is desirable, for example, by fractional crystallization, as will be exemplified hereinafter. In any event, the preferable amount of (III) with respect to (II) is a stoichiometric amount.

The amount of catalyst required to promote the addi tion of vinyl aromatic compound and methylene bis carbamate is not critical and can be varied widely. The amount is typically from 1 to 50 mole % and preferably about 15 to 25 mole %.

Preferably the catalyst is added dropwise to the warm reaction mixture containing the vinyl aromatic substrate. When the reaction is complete, the time generally being from about 8 hours to about 12 hours, the mixture is treated to remove or neutralize the catalyst by base. Then the unreacted materials and solvent, if used are removed, e.g., by distillation leaving the aromatic product of formula (IV) as a residue. Purification can be effected, e.g., by column separation using, e.g., methylene chloride, as an eluent.

In the hydrogenation step, hydrogen and a catalyst are used to produce the corresponding hexahydro and decahydro compound of formula (I) by standard procedures. For example, a solution of the compound of formula (IV) in an alcohol solvent, e.g., methanol or ethanol, is reacted at a suitable temperature, e.g., in the range of 30°C. to 170°C. and at an elevated pressure, e.g., 40 to 4,000 pounds per square inch of hydrogen with a catalyst, such as rhodium, ruthenium, rhenium, nickel, mixtures thereof, compounds thereof, and the like, until the aromatic rings are saturated. The products of formula (I) are isolated in conventional ways, e.g., by filtering off the catalyst and evaporating the solvent. If desired, any hydrogenated byproducts can be removed at this point, e.g., by fractional crystallization.

The cycloalkylalkyl bis urethanes (I) form the corresponding isocyanates (V) by thermal cracking while splitting off the corresponding alkanol. When styrene is used as the starting material, the ultimate

compound is of the formula:

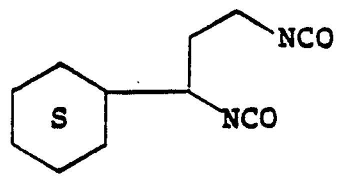

In many cases the alcohol, preferably methanol, can usefully be recycled with urea or isocyanic acid to form methyl carbamate which is then reacted with formaldehyde or a formaldehyde precursor to form compound (III).

In cracking the urethane esters (I) to form the corresponding isocyanates (V) the acidic catalyst must be removed or neutralized, for example, with calcium oxide, sodium carbamate, sodium hydroxide and the like, which is followed by cracking of the bis-urethane either solvent-free or in high boiling solvents, such as hexadecane, diphenyl ether, diisopropyl naphthalene and the like. Cracking takes place at temperatures on the order of 150°C to 700°C, preferably 400°C to 600°C splitting off the alcohol to yield the corresponding isocyanates. Pressures can vary widely, but it is convenient to use between about 30 and about 50 mm of mercury.

The urethanes produced in the invention have many uses. They can be used, for example as blocked isocyanates to cross link powder coatings and the like. In some instances herbicidal activity is anticipated. An important use is to produce the isocyanates of this invention, which are suitable to produce polyurethane film coatings, thick section reaction injection molded (RIM) articles and polyurethane forms, of improved light stability and low toxicity.

In accordance with known techniques coating compositions can be made by mixing polyols with effective amounts, 0.5 to 5 -NCO/-OH, of diisocyanates, optionally in a hydrocarbon solvent, preferably with a catalyst, e.g., 1 percent of a tin compound, and curing, e.g., at 60°-150°C for 1 minute to 1 hour. Hard, solvent-resistant films are thus obtained.

## DESCRIPTION OF THE PREFERRED EMBODIMENTS

The following examples illustrate the novel processes and novel compounds of the present invention. They are not to be construed to limit the claims in any manner whatsoever.

In the following Examples, the designations are used:

MEDU --methylene diurethane

SDU --1-phenyltrimethylene-1,3-diurethane

$H_6$-SDU - 1-cyclohexyltrimethylene-1,3-diurethane

$H_6$-SDI - 1-cyclohexyltrimethylene-1,3-diisocyanate

## EXAMPLE 1

(a) Preparation of DSU.

-To a flask containing 78 g of styrene and 162 g of MEDU in 300 g of toluene, 19.6 g of sulfuric acid catalyst (20 mole % based on styrene) was added over a period of 10 hours at 80°C. Analysis indicated that the products, obtained in 74% yield comprised equal weights of SDU and a byproduct of the formula

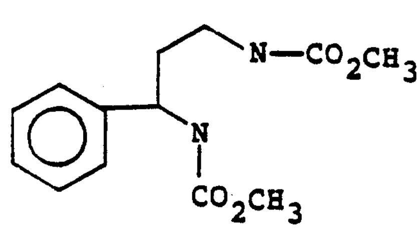

Pure SDU was isolated by column separation on silica gel absorbent, using methylene chloride as eluent.

(b) Preparation of $H_6$-SDU.

-The product of step (a) without separation of byproduct, 24.3g, was dissolved in 100 g of methanol and hydrogenated at 55 psi and 23°C using 4.86 g of Rh on carbon as a catalyst. $H_6$-SDU was obtained admixed with byproduct of the formula

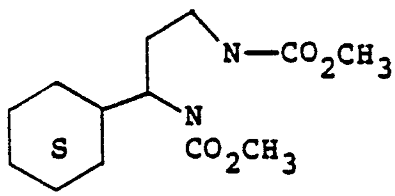

in quantitative yield. $H_6$-SDU was separated from the byproduct by fractional crystallization using a 99:1 mixture of hexane and methylene chloride as the solvents. $H_6$-SDU was a colorless solid, having a melting point of 107-108°C. It weighed 9.7 g, 40% theoretical yield.

In an alternative procedure, the pure SDU obtained by column separation was hydrogenated to yield pure $H_6$-SDU in a quantitative yield.

EXAMPLES 2-8

If the procedure of Example 1 is repeated using the following as starting materials:

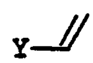

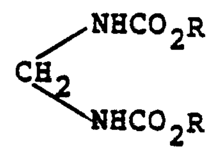

| Y | R |
| --- | --- |
| 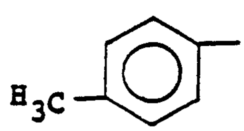 | $-CH_2CH_3$ |
| 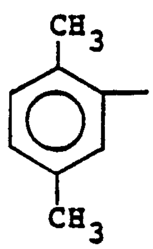 | $-CH_3$ |
| 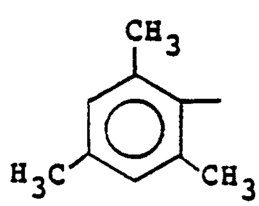 | $-CH_2(CH_2)_{16}CH_3$ |

| | |
|---|---|
| 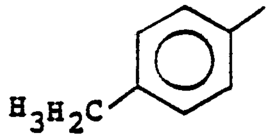 | $-CH_3$ |
| 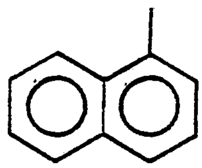 | $-CH_3$ |
| 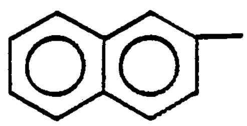 | $-CH_3$ |
| 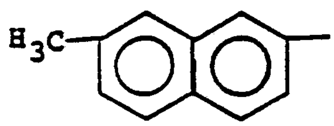 | $-CH_3$ |

the following diurethanes will be obtained

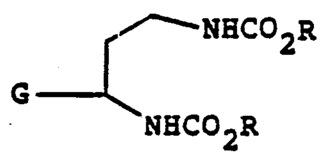

| Ex | G | R |
|---|---|---|
| 2 | 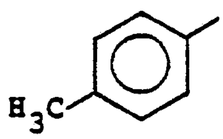 | $-CH_2CH_3$ |
| 3 | 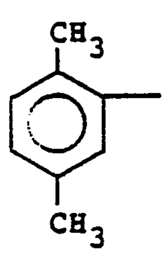 | $-CH_3$ |

| | | |
|---|---|---|
| 4 | 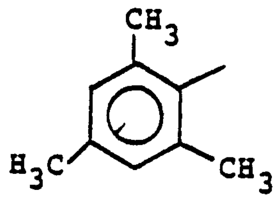 | $-CH_2(CH_2)_{16}CH_3$ |
| 5 | 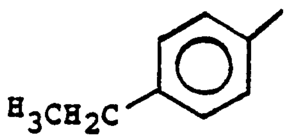 | $-CH_3$ |
| 6 | 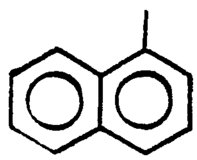 | $-CH_3$ |
| 7 | 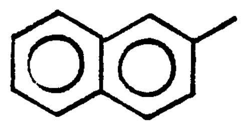 | $-CH_3$ |
| 8 | 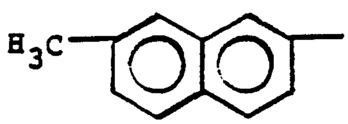 | $-CH_3$ |

EXAMPLE 9

Preparation of $H_6$-DSI.

-The diurethane $H_6$-SDU of Example 1 was thermally cracked at 500°C and 40 mm Hg pressure. Cracking was carried out in a 17 inch × 1-1/4 inch tube containing stainless steel packing (Propack 316-SS) using a nitrogen bleed and a feed rate of 5 g of $H_6$-SDU per hour. The yield of $H_6$-SDI from the reactor was 11 g, 85%. Distillation of this material gave very pure $H_6$-SDI, 98% by gas chromatography, and 100% by nuclear magnetic resonance spectroscopy. The product was a pale yellow liquid, b.p., 100°C/0.2 mm Hg, 145°C/4.0 mm Hg. It was soluble among others in toluene and methylene chloride.

EXAMPLES 10-16

If the procedure of Example 9 is repeated with the diurethanes of Examples 2-8, the following diisocyanates will be obtained:

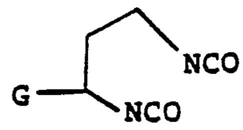

| Example | G |
| --- | --- |
| 10 | 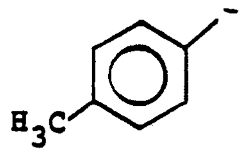 |
| 11 | 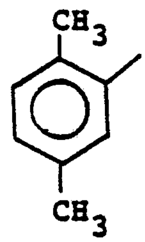 |
| 12 | 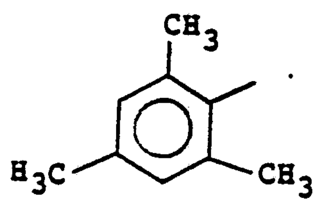 |
| 13 | 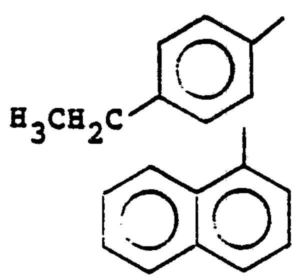 |
| 14 | |
| 15 | 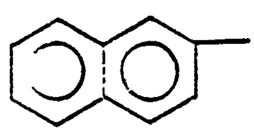 |
| 16 | 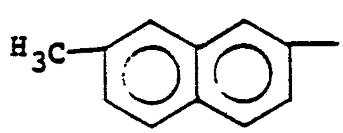 |

EXAMPLE 17

A curable composition is made comprising a hydroxy functional acrylic acid and the $H_6SDI$ of Example 9. A copolymer of hydroxyethyl acrylate with other acrylics (G-CURE® 867) and $H_6SDI$ at 50% non-volatiles in a hydrocarbon solvent, the -NCO/-OH ratio being 1.1/1.0 was treated with 1.0% (TRS) of a tin catalyst, UL-28, and spread on a 1200 S aluminum substrate and cured for 20 minutes at 60°, 80° and 100°C. All of the systems advanced within 1-4 days at room temperature to a hard, solvent resistant film.

The above-mentioned patents and publications are incorporated herein by reference.

Many variations will suggest themselves to those skilled in the art, in light of the above detailed description. For example, instead of using rhodium as a hydrogenation catalyst, ruthenium, activated nickel, and the like can be used. Instead of sulfuric acid catalyst, phosphoric acid and boron trifluoride-ether complex can be used. Instead of solvent-free cracking, cracking in hexadecane, a high boiling solvent can be used. Instead of using MEDU, a mixture of paraformaldehyde and methyl carbamate can be used. Instead of using $H_6SDI$, the diisocyanates of Examples 10-16 can be used. Instead of a hydroxy functional polyacrylate, a hydroxy functional polyester can be used. All such obvious variations are within the full intended scope of the appended claims.

## Claims

1. A process for the preparation of a diurethane of the formula:

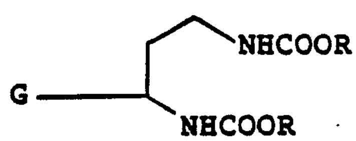

(I)

(I)

wherein G is selected from

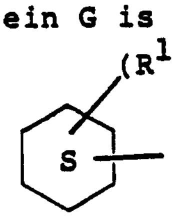

and

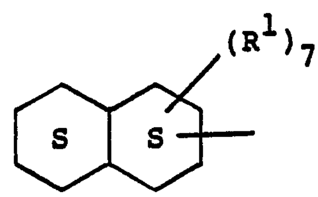

R is selected from alkyl of from about 1 to about 30 carbon atoms and $R^1$ is selected from hydrogen and alkyl of from about 1 to about 30 carbon atoms, said process comprising:

(a) reacting

(i) a vinyl aromatic hydrocarbon of the formula

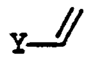 (II)

wherein Y is selected from

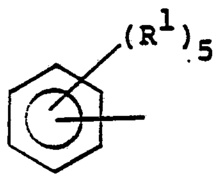 and 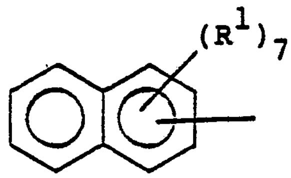 ,

with (ii) a methylene bis-alkylcarbamate of the formula

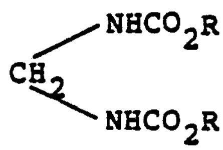 (III)

wherein R is as above defined, in the presence of

(iii) an effective catalytic amount of an acid at a temperature of from about 40°C to about 150°C until formation of a diurethane compound of the formula

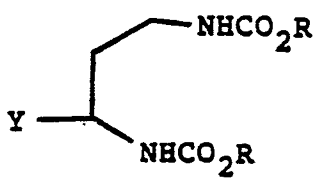 (IV)

wherein R and Y are as defined above is substantially complete, and

(b) catalytically hydrogenating the compound of formula (IV) until formation of the diurethane compound of formula (I) is substantially complete.

2. A process as defined in Claim 1 wherein the compound of formula (I) is

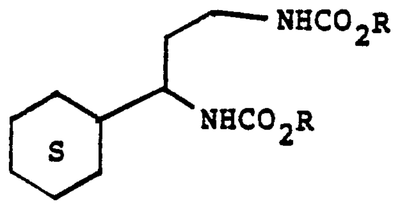

the compound of formula (II) is

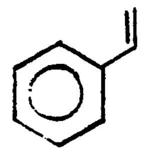

and the compound of formula (IV) is

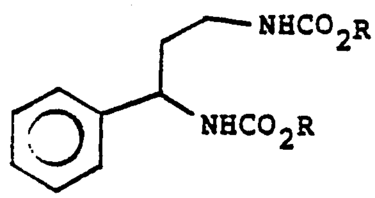

wherein R is alkyl of from about 1 to about 30 carbon atoms.

3. A process as defined in Claim 2 wherein R is $CH_3$.

4. A process as defined in Claim 1 wherein said acid catalyst (iii) is selected from sulfuric acid, phosphoric acid, hydrogen chloride, a sulfonic acid resin, hydrogen fluoride, alkyl sulfonic acid, a sulfate ester, a Lewis acid or a Bronsted acid.

5. A process as defined in Claim 4 wherein said acid catalyst (iii) is sulfuric acid.

6. A process according to Claim 1 wherein the catalyst for hydrogenation is selected from rhodium, ruthenium, nickel, or a compound thereof.

7. A process according to Claim 1 wherein step (b) is carried out at a temperature in the range of about 25°C to about 150°C and at a pressure in the range of from about 40 psi to about 3,000 psi.

8. A urethane compound of the formula:

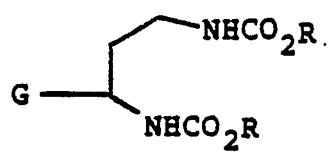

(I)

wherein G is selected from

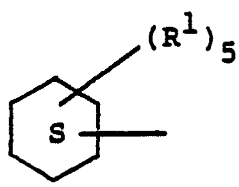

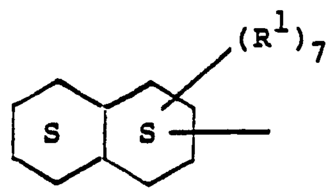

R is alkyl of from about 1 to about 30 carbon atoms and R' hydrogen or alkyl of from about 1 to about 30 carbon atoms.

9. A diurethane compound as defined in Claim 8 of the formula

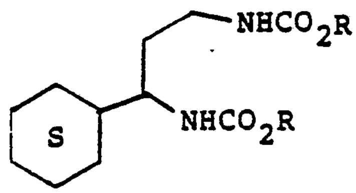

wherein R is alkyl of from about 1 to about 30 carbon atoms.

10. A dimethylurethane compound as defined in Claim 9 wherein R is $CH_3$.

11. A process for the preparation of an isocyanate compound of the formula:

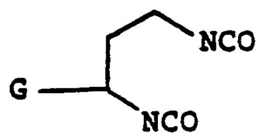

(V)

wherein G is selected from

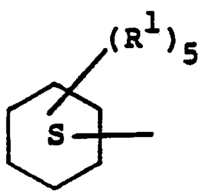

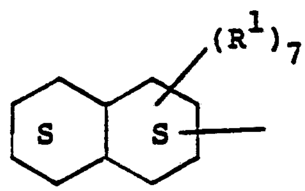

and $R^1$ is hydrogen or alkyl of from about 1 to about 30 carbon atoms, said process comprising heating a diurethane of the formula:

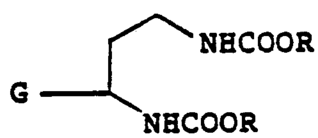

(I)

wherein G is as above defined and R is alkyl of from about 1 to about 30 carbon atoms, at a temperature of from about 150°C to about 700°C until formation of said diisocyanate compound of formula V is substantially complete.

12. A process as defined in Claim 11 wherein compound (V) is of the formula

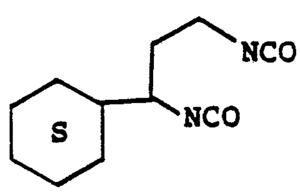

and compound (I) is of the formula

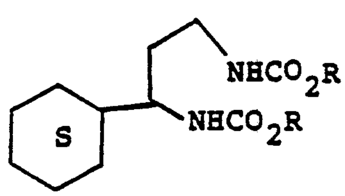

wherein R is alkyl of from about 1 to about 18 carbon atoms.

13. A process as defined in Claim 12 wherein R is $CH_3$.

14. A process as defined in Claim 11 wherein diurethane (I) is heated at a temperature of from about 400°C to about 600°C and at a pressure of from about 30 to about 50 mm of mercury.

15. A diisocyanate compound of the formulae:

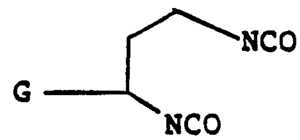

(V)

wherein G is selected from

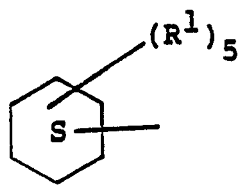

and

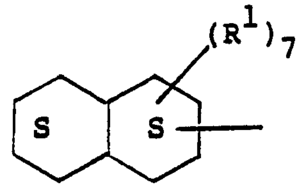

wherein $R^1$ is hydrogen or alkyl of from about 1 to about 3 carbon atoms.

16. A diisocyanate compound of the formula

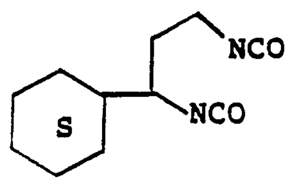

.

17. A curable composition comprising:

(i) a polyol; and

(ii) a diisocyanate of the formula as defined Claim 15.

18. A curable composition comprising:

(i) a hydroxy functional polyacrylate; and

(ii) a diisocyanate as defined in Claim 16.